# EUROPÄISCHE PATENTSCHRIFT

(11) **EP 0 755 633 B1**
(45) Veröffentlichungstag und Bekanntmachung des Hinweises auf die Patenterteilung: **27.10.1999**
(21) Anmeldenummer: 95111978.3
(22) Anmeldetag: 28.07.1995
(51) Int. Cl.: A23L 1/30, A23L 1/305, A23L 1/302, A23L 1/304, A61K 31/20

(54) **Zwei-Phasen-Präparat**
Two phases preparation
Préparation en deux phases

(43) Veröffentlichungstag der Anmeldung: 29.01.1997
(73) Patentinhaber: Schlachter, Herbert, 81543 München (DE); Hamm, Michael, Prof. Dr., 20148 Hamburg (DE)
(72) Erfinder: Schlachter, Herbert, 81543 München (DE); Hamm, Michael, Prof. Dr., 20148 Hamburg (DE)
(74) Vertreter: VOSSIUS & PARTNER

(56) Entgegenhaltungen:
- EP-A- 0 146 474
- EP-A- 0 216 419
- EP-A- 0 224 157
- EP-A- 0 296 751
- EP-A- 0 366 480
- EP-A- 0 409 559
- FR-A- 2 394 292
- DATABASE WPI Section Ch, Week 8840 Derwent Publications Ltd., London, GB; Class B05, AN 82-60170E XP002008863 & JP 57 093 913 A (AJINOMOTO) , 11.Juni 1982

## Beschreibung

Die Erfindung betrifft die Verwendung eines Produkts A und eines Produkts B für die Herstellung eines Nahrvngsergänzungsmittel-Zwei-Phasen-Präparats zur zeitlich abgestuften Anwendung, das ein Produkt A für die Tagesphase und ein Produkt B für die Nachtphase umfaßt, welche jeweils unabhängig voneinander mindestens einen Vertreter der ungesättigten Fettsäuren und/oder mindestens einen Vertreter aus der Gruppe der Spurenelemente und Mineralstoffe und/oder mindestens einen Vertreter aus der Gruppe der Vitamine und/oder mindestens einen Vertreter aus der Gruppe der bioaktiven Pflanzensubstanzen, z.B. Polyphenole, Bioflavonoide oder Ballaststoffe, und/oder mindestens eine Aminosäure und/oder ein Aminosäurederivat umfassen, wobei das Produkt A und/oder das Produkt B gegebenenfalls zusätzlich Sojalecithin enthalten kann, mit der Maßgabe, daß sich Produkt A und Produkt B in ihrer mengenmäßigen und/oder ihrer stofflichen Zusammensetzung voneinander unterscheiden. Das Präparat kann als Nahrungsergänzungsmittel (Diätetikum) verwendet werden.

Insbesondere betrifft die Erfindung die Verwendung eines Produkts A und eines Produkts B für die Herstellung eines Nahrungsergänzungsmittel-Zwei-Phasen-Präparats zur zeitlich abgestuften Anwendung, das ein Produkt A und ein Produkt B umfaßt, welche jeweils unabhängig voneinander mindestens einen Vertreter der ungesättigten Fettsäuren, einen Vertreter aus der Gruppe der Spurenelemente und Mineralstoffe und einen Vertreter aus der Gruppe der Vitamine umfassen, wobei das Produkt A und/oder das Produkt B gegebenenfalls zusätzlich Sojalecithin und/oder mindestens einen Vertreter aus der Gruppe der bioaktiven Pflanzensubstanzen und/oder mindestens eine Aminosäure und/oder ein Aminosäurederivat enthalten kann, das als Nahrungsergänzungsmittel (Diätetikum) verwendet wird.

Es ist allgemein bekannt, daß die Nährstoffe Kohlenhydrate sowie die Nahrungsfette vorrangig zur Deckung des Energiebedarfs dienen. Eiweiß bzw. Protein ist ein wichtiger Aufbaustoff für Zellen und körpereigene Wirkstoffe, wie Enzyme und bestimmte Hormone. Zunehmend gewinnen die mehrfach ungesättigten Fettsäuren, Vitamine, Mineralstoffe und Spurenelemente und die bioaktiven Pflanzenstoffe wie die Flavonoide für die Gesundheit und Leistungsfähigkeit an Bedeutung.

Vitamine, Mineralstoffe und Spurenelemente, mehrfach ungesättigte Fettsäuren und bioaktive Pflanzenstoffe wie z.B. Flavonoide sind lebensnotwendige Reglerstoffe im Stoffwechsel und Schutznährstoffe für die Gesundheit. Einseitige Ernährung, strenge Schlankheitsdiäten, Bevorzugung von kalorienreichen Lebensmitteln mit einer niedrigen Nährstoffdichte, ein hoher Verarbeitungsgrad vieler Nahrungsmittel, aber auch die teilweise Verarmung der Böden an wichtigen Mineralstoffen erschweren jedoch eine optimale Versorgung mit allen benötigten Nährstoffen. Hinzu kommt, daß Umweltbelastungen, Rauchen, Alkohol und Medikamente sowie Streß den Bedarf an einigen lebensnotwendigen Nährstoffen, z.B. Magnesium, Zink, Vitamin E, Vitamin C, Vitamine der B-Gruppe und Beta-Carotin, erhöhen. Eine nahrungsergänzende Nährstoffsubstitution trägt daher zur sicheren Nährstoffversorgung und zum optimalen Gesundheitsschutz sowie zum Wohlbefinden bei, ebenso wie eine ausgewogene Ernährung, die ballaststoffreich und fettarm ist. Empfehlungen für die Nährstoffversorgung sind beispielsweise beschrieben in Deutsche Gesellschaft für Ernährung, "Empfehlungen für die Nährstoffzufuhr", 5. Auflage (1991), Umschau-Verlag, Frankfurt/Main".

Vitamine sind lebensnotwendige Nahrungsbestandteile, die für die normalen Funktionen heterotropher Lebewesen mehr oder weniger obligat und bedarfsgerecht zuzuführen sind, da sie nur aus äußeren Quellen bzw. unter dem Einfluß von Milieufaktoren (z.B. Darmflora) zugänglich sind. Ihre spezifische biokatalytische Wirkung beruht auf Ersatz der dem metabolischen Verschleiß unterliegenden Wirkgruppen von Enzymen. Von wissenschaftlicher Seite her ist z.B. bekannt, daß die Vitamine der B-Gruppe als Coenzyme am intermediären Stoffwechsel beteiligt sind und die Vitamine C, E und β-Carotin vor allem als Antioxidantien wirken. Mangel infolge ungenügender Zufuhr oder Resorption, Störungen der Darmflora oder Metabolismus, Antivitamin-Einwirkung oder gesteigerter Verbrauch führen zu Hypo- und Avitaminosen.

Mineralstoffe und Spurenelemente sind ferner lebensnotwendige Reglerstoffe im Stoffwechsel. Calcium ist der wichtigste mineralische Aufbaustoff für Knochen und Zähne. Eine gute Calciumversorgung bis zum 30. Lebensjahr ist der beste Osteoporoseschutz im Alter. Danach sorgt genügend Nahrungscalcium dafür, daß die Knochenspeicher nicht unnötig strapaziert werden. Calcium ist für Frauen oft ein kritischer Nährstoff. Zink, Magnesium und die Vitamine der B-Gruppe sind Hochleistungselemente, indem sie Enzyme aktivieren und den Stoffwechsel von Kohlenhydraten, Fetten und Eiweißstoffen erst ermöglichen. Silicium wirkt sich günstig auf Stabilität und Erhalt von Haut, Haaren und Nägeln aus. Ferner ist wissenschaftlich unbestritten, daß Zink eine essentielle Funktion im Immunsystem und im Stoffwechsel der Haut hat.

Mehrfach ungesättigte Fettsäuren aus rein pflanzlichen Quellen, wie z.B. Nachtkerzenöl und Leinöl, enthalten Linolsäure und α- und γ-Linolensäure, die wichtige Ausgangssubstanzen für biologisch aktive Reglersubstanzen, wie z.B. Eicosanoide und Prostaglandine, im Stoffwechsel sind und die für ein gesundes Gleichgewicht im Stoffwechsel sorgen. Eicosanoide und Prostaglandine, auch als Gewebshormone bezeichnet, werden von Wissenschaftlern in bezug auf ihre gesundheitsstabilisierende Wirkung zur Zeit intensiv erforscht. Bekannt ist der günstige Einfluß der mehrfach ungesättigten Fettsäuren auf den Cholesterinstoffwechsel und die gesunde Hautfunktion sowie bei entzündlichen Vorgängen. Ferner ist bekannt, daß mehrfach ungesättigte Fettsäuren vom Typ der ω-3-(Eicosapentaensäure, α-Linolensäure) und ω-6-Fettsäuren (Linolsäure, γ-Linolensäure) günstige Effekte bei Migräne, Rheuma, Neurodermitis, Psoriasis, Prämenstruellem Syndrom sowie bei belastungsinduzierten Regenerationsprozessen im Sport haben (vgl. A. Berg, D. König, H. Schlachter, J. Keul, Deutsche Zeitschrift für Sportmedizin, Jahrg. 44 (1993), Sonderheft und darin zitierte Literatur).

Karotten-, Brokkoli- und Citrusextrakte sind natürliche Vitamin- und Mineralstoffquellen und enthalten die sogenannten sekundären Pflanzenstoffe, fachwissenschaftlich auch bioaktive Pflanzenstoffe genannt, deren Bedeutung für die Gesundheit erkannt wurde und zur Zeit ebenfalls erforscht wird. Zu diesen natürlichen Pflanzenstoffen zählen auch die Bioflavonoide, die die Wirkung von Vitamin C in bezug auf die Abwehrkräfte, die Gefäßwände und das Bindegewebe wirksam unterstützen. Ferner ist bekannt, daß die Bioflavonoide antioxidative Eigenschaften haben und damit die Wirkung der Vitamine C, E und Beta-Carotin synergistisch ergänzen. Auch grüner Tee ist eine reiche Quelle für diese pflanzlichen Antioxidantien (z.B. Polyphenole) (vgl. B. Watzl, C. Leitzmann, "Bioaktive Substanzen in Lebensmitteln", Hippokrates Verlag, Stuttgart, 1995).

Aminosäuren sind neben ihrer Funktion als Proteinbausteine Vorstufen von biologisch wirksamen Verbindungen, z.B. Serotonin, Wachstums- und Streßhormonen. Es werden ihnen auch antikatabole Eigenschaften zugeschrieben (vgl. hierzu K.R. Geiß, M. Hamm, "Handbuch Sporternährung", Rowohlt Verlag, 1992).

Im Handel sind zahlreiche Nahrungsergänzungsmittel erhältlich, die Vitamine einzeln (z.B. Vitamin E-Präparate) oder in Kombination (z.B. Multivitamin-Präparate) und/oder Mineralstoffe und Spurenelemente einzeln (z.B. Magnesium- oder Eisen-Präparate) oder in Kombination (z.B. Multi-Mineral-Kapseln) enthalten. Ebenso gibt es Präparate mit mehrfach ungesättigten Fettsäuren allein oder in Kombination mit Vitaminen und Mineralstoffen. Beispielsweise umfaßt ein Präparat viele unterschiedliche Vitamine und einige ausgewählte Mineralstoffe, die den Tagesbedarf der enthaltenen Nährstoffe decken.

Ein weiteres im Handel befindliches Präparat ist charakterisiert als ergänzende bilanzierte Diät zur Deckung eines spezifisch erhöhten Bedarfs essentieller Nährstoffe, insbesondere im Hinblick auf die zielgerichtete Ernährung, z.B. nach Herzinfarkt oder Bypass-Operationen, wo die angespannte Stoffwechsellage eine Kompensation erfordert. Dieses sogenannte diätische Lebensmittel umfaßt für den Tagesbedarf einen Beutel mit 9 Tabletten bzw. Kapseln, wobei in der Regel der Inhalt eines Beutels zu oder nach den Mahlzeiten eingenommen wird, es aber auch möglich ist, die Einnahme der einzelnen Kapseln bzw. Tabletten in beliebiger Reihenfolge auf die einzelnen Mahlzeiten zu verteilen. Der Tagesbedarf dieses Präparats besteht aus lebensnotwendigen Vitaminen, wie z.B. den Vitaminen A, B1, B2, B3, B6, B12, C, E, D3, K und Beta-Carotin, Spurenelementen, wie z.B. Eisen, Zink, Mangan, Chrom, Magnesium und Selen, und speziellen essentiellen Fettsäuren. Dabei weist dieses diätische Lebensmittel höhere Mengen an Vitaminen auf, als für die tägliche Zufuhr bei gesunden Erwachsenen grundsätzlich empfohlen wird.

Die meisten der im Handel befindlichen Nahrungsergänzungsmittel liegen in Form von Kapseln oder Tabletten vor und werden in der Regel Tageszeit-unabhängig eingenommen.

Bisher haben sich Nahrungsergänzungen nur allgemein auf die Deckung des Nährstoffbedarfs bei unzureichender Ernährung bezogen. Trotz der Vielzahl an Nahrungsergänzungsmitteln ist bis jetzt noch keine optimale Lösung für eine angemessene Versorgung angeboten worden, die die wissenschaftlichen Erkenntnisse ausreichend berücksichtigt. Von wissenschaftlicher Seite her ist bekannt, daß im Gegensatz zu einer Kombination vieler Vitamine und Mineralstoffe in einer einzigen Darreichungsform eine verteilte Gabe dieser Stoffe physiologisch sinnvoller ist. Im Vergleich zu einer hohen Nährstoffdosierung weisen die geringeren Einzeldosen eine bessere Verfügbarkeit auf. Ebenso ist bekannt, daß die gleichzeitige Aufnahme von Mineralstoffen und Spurenelementen in einer einzelnen Darreichungsform die Resorption von Einzelkomponenten ungünstig beeinflussen kann. Davon sind u.a. Magnesium und Calcium sowie Eisen, Zink und Selen untereinander betroffen.

In vielen bekannten Präparaten ist die Zusammensetzung der Inhaltsstoffe nicht immer sinnvoll, weil die gleichzeitige Gabe aller essentiellen Nährstoffe die Verfügbarkeit einzelner Bestandteile verringern kann (z.B. wird Magnesium schlechter resorbiert, wenn der Calciumgehalt in einer Einzelgabe deutlich höher als der Magnesiumgehalt ist). Auch zwischen Eisen und mehrfach ungesättigten Fettsäuren können aufgrund der prooxidativen Eigenschaften von Eisen unerwünschte Reaktionen auftreten.

In einem der vorstehend genannten, im Handel befindlichen Präparate muß der Anwender am Tag neun Kapseln bzw. Tabletten zu einer oder mehreren Mahlzeiten einnehmen. Eine für den Anwender akzeptable Tagesdosierung sollte jedoch maximal 3 Einzeldosierungen pro Tag (z.B. 1 bis 3 Kapseln pro Tag) enthalten.

Der menschliche Stoffwechsel weist Tages- und Nachtphasen auf. Es wird angenommen, daß diese Tages- und Nachtphasen auch einen Einfluß auf Nährstoffhaushalt, Nährstoffaufnahme und -bedarf des Körpers haben. Eine Aufgabe der Erfindung ist es, ein sinnvoll zusammengesetztes Zwei-Phasen-Präparat bereitzustellen, in welchem die tägliche Nährstoffgabe zeitlich im Hinblick auf aktivierende Nährstoffprinzipien auf die erste Tageshälfte und im Hinblick auf regenerative Nährstoffkombinationen auf die zweite Tageshälfte verteilt wird. Eine weitere Aufgabe der vorliegenden Erfindung ist, ein Zwei-Phasen-Präparat bereitzustellen, das auf die jeweiligen Ansprüche bestimmter Zielgruppen ausgerichtet ist. Das Zwei-Phasen-Präparat soll als Diätetikum anwendbar sein.

Eine Aufgabe der vorliegenden Erfindung wird gelöst durch die Verwendung eines Produkts A und eines Produkts B für die Herstellung eines Nahrungsergänzungsmittel-Zwei-Phasen-Präparats zur zeitlich abgestuften Anwendung, umfassend ein Produkt A für die Tagesphase (erste Phase, aktive Phase) und ein Produkt B für die Nachtphase (zweite Phase, regenerative Phase), wobei das Produkt A
a) mindestens einen Vertreter der ungesättigten Fettsäuren und/oder
b) mindestens einen Vertreter aus der Gruppe Spurenelemente und Mineralstoffe und/oder
c) mindestens einen Vertreter aus der Gruppe der Vitamine und/oder
d) mindestens einen Vertreter der bioaktiven Pflanzenstoffe und/oder
e) mindestens eine Aminosäure und/oder ein Aminosäurederivat,
und
das Produkt B
a) mindestens einen Vertreter der ungesättigten Fettsäuren und/oder
b) mindestens einen Vertreter aus der Gruppe Spurenelemente und Mineralstoffe und/oder
c) mindestens einen Vertreter aus der Gruppe der Vitamine und/oder
d) mindestens einen Vertreter der bioaktiven Pflanzenstoffe und/oder
e) mindestens eine Aminosäure und/oder ein Aminosäurederivat,
umfaßt, mit der Maßgabe, daß sich Produkt A und Produkt B in ihrer mengenmäßigen und/oder ihrer stofflichen Zusammensetzung voneinander unterscheiden.

Die vorliegende Erfindung betrifft in einer bevorzugten Ausführungsform die Verwendung eines Produkts A und eines Produkts B für die Herstellung eines Nahrungsergänzungsmittel-Zwei-Phasen-Präparats zur zeitlich abgestuften Anwendung, umfassend ein Produkt A für die erste Phase und ein Produkt B für die zweite Phase, wobei das Produkt A
a) mindestens einen Vertreter der ungesättigten Fettsäuren,
b) mindestens einen Vertreter aus der Gruppe Spurenelemente und Mineralstoffe und
c) mindestens einen Vertreter aus der Gruppe der Vitamine,
und
das Produkt B
a) mindestens einen Vertreter der ungesättigten Fettsäuren,
b) mindestens einen Vertreter aus der Gruppe Spurenelemente und Mineralstoffe und
c) mindestens einen Vertreter aus der Gruppe der Vitamine umfaßt.

In einer weiteren bevorzugten Ausführungsform können das Produkt A und/oder das Produkt B zusätzlich Sojalecithin umfassen. Sojalecithin wird dabei bevorzugt in einem Mengenanteil von 1 bis 40 Gew.-%, besonders bevorzugt von 5 bis 20 Gew.-% zugegeben, bezogen auf die Summe aller Wirkstoffe des Produkts A bzw. B.

In einer weiteren Ausführungsform können das Produkt A und/oder das Produkt B der bevorzugten Ausführungsformen zusätzlich mindestens einen Vertrete aus der Gruppe der bioaktiven Pflanzenstoffe umfassen.

In einer weiteren Ausführungsform können das Produkt A und/oder das Produkt B der bevorzugten Ausführungsformen zusätzlich Aminosäuren und/oder Aminosäurederivate, auch in Kombination mit anderen Wirkstoffen der vorliegenden Erfindung, umfassen.

Der Ausdruck "zeitlich abgestufte Anwendung" bedeutet, daß das Produkt A zu Beginn der Tagesphase oder aktiven Phase und das Produkt B zu Beginn der Nachtphase oder regenerativen Phase des menschlichen Stoffwechsels angewendet werden bzw. seine Wirkung bereitstellen soll. Bevorzugt wird das Produkt A am Morgen und das Produkt B am Abend eines Tages angewendet.

Der Ausdruck "Zwei-Phasen" bezieht sich auf die Tages- oder aktive Phase als erste Phase (erste Tageshälfte) und auf die Nacht- oder regenerative Phase als zweite Phase (zweite Tageshälfte) des menschlichen Stoffwechsels.

Das Produkt A und das Produkt B der vorliegenden Erfindung unterscheiden sich voneinander in ihrer mengenmäßigen und/oder ihrer stofflichen Zusammensetzung.

Die Rezepturen der vorliegenden Erfindung berücksichtigen bevorzugt synergistische Effekte, z.B. bei Vitamin C und Bioflavonoiden sowie bei Vitamin C und mehrfach ungesättigten Fettsäuren.

Im Zwei-Phasen-Präparat der vorliegenden Erfindung können die Produkte A und B alle bekannten Vertreter der Vitamine umfassen. Insbesondere werden im Zwei-Phasen-Präparat bevorzugt für das Produkt A die Vitamine C und E als Antioxidantien (Zellschutzvitamine), sowie die Vitamine B1, B2, B6, B12, Pantothensäure, Beta-Carotin und Niacin, und für das Produkt B bevorzugt die Vitamine B1, B2, B6, B12, Pantothensäure, Niacin und Folsäure und Biotin als Stoffwechselregler im Sinne von Coenzymen des Kohlenhydrat-, Fett- und Eiweißstoffwechsels verwendet.

Der Anteil der Vitamine beträgt in den bevorzugten Ausführungsformen des Zwei-Phasen-Präparats der vorliegenden Erfindung bevorzugt im Produkt A 1 bis 70 Gew.-%, im Produkt B 0,5 bis 50 Gew.-%, bezogen auf die Summe aller Wirkstoffe des Produkts A bzw. B. In einer stärker bevorzugten Ausführungsform umfaßt das Produkt A 5 bis 50 Gew.-% Vitamine, insbesondere bevorzugt 20 bis 45 Gew.-%, und das Produkt B 1 bis 40 Gew.-% Vitamine, insbesondere bevorzugt 1 bis 35 Gew.-%. Die Vitamine werden sowohl in natürlicher Form als Extrakte (z.B. D-α-Tocopherol) als auch synthetisch (z.B. B-Vitamine) zugesetzt, wobei Unterschiede in der Vitaminwirksamkeit damit nicht verbunden sind.

Vertreter aus der Gruppe der Spurenelemente und Mineralstoffe für das Zwei-Phasen-Präparat der vorliegenden Erfindung sind alle bekannten Mineralstoffe und Spurenelemente. In einer bevorzugten Ausführungsform umfaßt das Produkt A des Zwei-Phasen-Präparats insbesondere Zink, Magnesium und Chrom und das Produkt B Silicium, Magnesium, Zink und Calcium. Der Anteil der Mineralstoffe und Spurenelemente in den bevorzugten Ausführungsformen beträgt dabei bevorzugt im Produkt A und im Produkt B 1 bis 80 Gew.-%, stärker bevorzugt im Produkt A 2 bis 50 Gew.-%, insbesondere bevorzugt 5 bis 30 Gew.-%, und im Produkt B 2 bis 70 Gew.-%, insbesondere bevorzugt 3 bis 60 Gew.-%, bezogen auf die Summe aller Wirkstoffe im Produkt A bzw. B. Die Spurenelemente werden bevorzugt in Form von Chrom-, Zink- und Siliciumhefe oder anderen organischen Verbindungen zugesetzt. Die Mengenelemente, wie z.B. Magnesium und Calcium, liegen als anorganische Salzverbindungen oder organische Verbindungen vor. Beispielsweise wird Magnesium oder Calcium in Form von Magnesium- oder Calciumcarbonat zugegeben.

Im Zwei-Phasen-Präparat der vorliegenden Erfindung können alle bekannten ungesättigten Fettsäuren verwendet werden. Es werden bevorzugt die in pflanzlichen und tierischen Ölen enthaltenen ungesättigten Fettsäuren verwendet. Mehrfach ungesättigte Fettsäuren aus pflanzlichen und tierischen Quellen sind essentielle Vorstufen von wichtigen Stoffwechselregulatoren (Eicosanoide und Prostaglandine). Die ungesättigten Fettsäuren sind dabei in den bevorzugten Ausführungsformen des Zwei-Phasen-Präparats im Produkt A bevorzugt in einem Anteil von 5 bis 90 Gew.-%, stärker bevorzugt 10 bis 70 Gew.-%, insbesondere bevorzugt 30 bis 60 Gew.-%, und im Produkt B in einer bevorzugten Menge von 1 bis 90 Gew.-%, stärker bevorzugt 3 bis 85 Gew.-%, insbesondere bevorzugt 5 bis 80 Gew.-%, vorhanden, bezogen auf die Summe aller Wirkstoffe im Produkt A bzw. B. Beispiele für bevorzugte rein pflanzliche Quellen von ungesättigten Fettsäuren sind Nachtkerzenöl, Leinöl, Olivenöl und Weizenkeimöl und bevorzugte tierische Quellen sind z.B. Fischöl.

Gegebenenfalls kann das Zwei-Phasen-Präparat der vorliegenden Erfindung alle bekannten bioaktiven Pflanzenstoffe enthalten. Die in der vorliegenden Erfindung verwendeten bioaktiven Pflanzensubstanzen umfassen insbesondere Carotinoide, Phytosterine, Saponine, Polyphenole, Flavonoide, Terpene, Phytoöstrogene, Sulfide, Phytinsäure und Ballaststoffe. Von den vorstehend genannten bioaktiven Pflanzensubstanzen werden in der vorliegenden Erfindung besonders bevorzugt die Polyphenole, die Flavonoide und Bioflavonoide und die Ballaststoffe verwendet. Besonders bevorzugt werden im Zwei-Phasen-Präparat Bioflavonoide verwendet, wie z.B. Citrusfrüchte-Flavonoide, die natürlichen Ursprungs sind. Die bioaktiven Pflanzenstoffe können dabei in den bevorzugten Ausführungsformen im Produkt A und/oder im Produkt B des Zwei-Phasen-Präparats in einer bevorzugten Menge von 1 bis 50 Gew.-%, stärker bevorzugt 1 bis 30 Gew.-%, insbesondere bevorzugt 1 bis 20 Gew.-% vorhanden sein, bezogen auf die Summe aller Wirkstoffe im Produkt A bzw. B. Bevorzugte natürliche Quellen für die gefäßwirksamen und antioxidativen Bioflavonoide sind Karotten-, Brokkoli- und Citrusextrakte sowie grüner Tee.

Das Zwei-Phasen-Präparat der vorliegenden Erfindung kann gegebenenfalls alle bekannten Aminosäuren und Aminosäurederivate enthalten. Bevorzugt werden im Zwei-Phasen-Präparat Aminosäure- und/oder Aminosäurederivatemischungen verwendet, umfassend die Aminosäuren Arginin, Ornithin, Leucin, Isoleucin, Valin und Tryptophan, und die Aminosäurederivate Taurin und Carnitin. Weiter bevorzugt sind Aminosäure- und Aminosäurederivatemischungen mit Vitamin B6 und Coenzym Q₁₀ zur Förderung der Regeneration und des Aufbaustoffwechsels in der Nachtphase. Die Aminosäuren können einzeln oder in Form von Mischungen in den bevorzugten Ausführungsformen im Produkt A und/oder im Produkt B des Zwei-Phasen-Präparats in einer bevorzugten Menge von 10 bis 95 Gew.-%, stärker bevorzugt 50 bis 90 Gew.-%, insbesondere bevorzugt 80 bis 90 Gew.-%, vorhanden sein, bezogen auf die Summe aller Wirkstoffe im Produkt A bzw. B. Die Aminosäuren und ihre Derivate werden bevorzugt in reiner Form zugegeben.

Gegebenenfalls kann das Zwei-Phasen-Präparat der vorliegenden Erfindung alle bekannten Hilfs-, Zusatz- und Trägerstoffe und Lösungsmittel umfassen. Bevorzugte Beispiele sind dabei Milchfett, un-, teil- oder hydriertes Sojafett, Sojaöl, Walnußbutter, Glycerin, Gelatine, Sorbit-Lösung oder -Trockensubstanz, Eisenoxid, Titandioxid, Patentblau, Chinolingelb, Ponceaue und Wasser.

Die Herstellung des Zwei-Phasen-Präparats der vorliegenden Erfindung kann in jedem Fachmann geläufiger Weise dadurch erfolgen, daß man die Wirkstoffe zusammen mit geeigneten, nicht-toxischen, inerten, pharmazeutisch verträglichen festen oder flüssigen Trägermaterialien und gegebenenfalls den üblichen Zusatz- und Hilfsstoffen und Lösungsmittel in eine galenische Darreichungsform bringt. Verfahren zur Herstellung galenischer Darreichungsformen, wie z.B. Weichgelatinekapseln, sind beispielsweise beschrieben in H. Sucker, P. Fuchs, P. Speiser, "Pharmazeutische Technologie", 2. Aufl. (1991), Georg Thieme Verlag, Stuttgart; R. Voigt, "Lehrbuch der pharmazeutischen Technologie", Chemie Verlag, 1976; Fahrig und Hofer, "Die Kapsel", Wissenschaftliche Verlagsgesellschaft, 1982; Remington's Pharmaceutical Sciences, 15th Edition (1975), Mack Publishing Company, Easton, Pennsylvania.

Für das Zwei-Phasen-Präparat der vorliegenden Erfindung sind alle bekannten Applikationsformen, wie z.B. Kapseln, Tabletten, Dragees, Lösungen etc., und Applikationswege möglich. Insbesondere wird jedoch die orale Verabreichung bevorzugt, wobei bevorzugte Applikationsformen Weichgelatinekapseln, Trinkampullen, kleine Beutel (Amphiolen) und Fertiggetränke sind. Werden beispielsweise für das Zwei-Phasen-Präparat der vorliegenden Erfindung Weichgelatinekapseln verwendet, so erfolgt die Dosierung bevorzugt derart, daß das Produkt A, welches die sinnvollen Nährstoffe für die Tagesphase oder aktive Phase enthält, in eine Kapsel A eingeschlossen wird, welche bevorzugt morgens verabreicht werden soll. Das Produkt B, das die sinnvollen Nährstoffe für die Nacht- bzw. regenerative Phase enthält, wird in eine Kapsel B eingeschlossen, welche bevorzugt abends verabreicht werden soll. Dabei enthält jeweils die Kapsel A bzw. B die Gesamtdosis für die Tagesphase bzw. für die Nachtphase eines Tages. Es wird empfohlen, pro Tag morgens eine Kapsel A und abends eine Kapsel B zu verabreichen. Das Zwei-Phasen-Präparat der vorliegenden Erfindung kann als Kit zur Verfügung gestellt werden, bestehend beispielsweise aus einer gleichen Anzahl von Kapseln A und Kapseln B. Weitere Applikationsformen können Trinkampullen und Beutel sein. Es ist jedoch auch möglich, Produkt A und Produkt B in einer einzigen Applikationsform derart modifiziert bereitzustellen, daß eine zeitlich sich voneinander unterscheidende Wirkstofffreigabe von Produkt A und Produkt B erzielt wird (z.B. Depotform).

Die vorliegende Erfindung wird anhand von Beispielen weiter erläutert.

### Beispiel 1

Dieses Beispiel umfaßt ein Zwei-Phasen-Präparat zur zeitlich abgestuften Anwendung, das speziell auf den Organismus der Frau abgestimmt ist, als Diätetikum. Die in Tabelle I angegebenen Zusammensetzungen für die Kapsel A bzw. für die Kapsel B wurden durch dem Fachmann geläufige Verfahren erhalten und auf üblichem Wege in Weichgelatinekapseln eingeschlossen. Zusätzlich kann die Rezeptur bekannte Zusatz-, Hilfs- und Trägerstoffe und Lösungsmittel enthalten.

**Tabelle I**

| Kapsel A | |
|---|---|
| 10 mg | Karottenextrakt |
| 200 mg | Nachtkerzenöl |
| 100 mg | Leinöl |
| 50 mg | Sojalecithin |
| 5 mg | Beta-Carotin in Form einer 30%igen |
| | Beta-Carotin-Suspension |
| 30 mg | Vitamin E |
| 200 mg | Vitamin C |
| 200 mg | Zinkhefe (10 mg Zn/g Hefe) |
| 50 mg | Magnesium |
| 50 mg | Flavonoide aus Citrusfrüchten |

| Kapsel B: | |
|---|---|
| 20 mg | Brokkoliextrakt |
| 200 mg | Nachtkerzenöl |
| 100 mg | Leinöl |
| 5 mg | Vitamin E |
| 100 mg | Siliciumhefe (30 mg Si/g Hefe) |
| 300 mg | Calcium |
| 3 mg | Vitamin B1 |
| 3 mg | Vitamin B2 |
| 5 mg | Vitamin B6 |
| 8 mg | Pantothensäure |
| 10 mg | Niacin |
| 5 µg | Vitamin B12 |
| 150 µg | Folsäure |
| 100 µg | Biotin |
| 100 mg | Zinkhefe (10 mg Zn/g Hefe) |
| 50 mg | Flavonoide aus Citrusfrüchten |

### Beispiel 2

Dieses Beispiel umfaßt ein Zwei-Phasen-Präparat zur zeitlich abgestuften Anwendung, speziell auf den Organismus des Mannes abgestimmt, als Diätetikum. Die in Tabelle II angegebenen Zusammensetzungen für die Kapsel A bzw. für die Kapsel B wurden durch dem Fachmann geläufige Verfahren erhalten und auf üblichem Wege in Weichgelatinekapseln eingeschlossen. Zusätzlich kann die Rezeptur bekannte Zusatz-, Hilfs- und Trägerstoffe und Lösungsmittel enthalten.

**Tabelle II**

| Kapsel A: | |
|---|---|
| 300 mg | Fischöl (Eicosapentaensäure) |
| 100 mg | Leinöl |
| 100 mg | Sojalecithin |
| 36 mg | Vitamin E |
| 200 mg | Vitamin C |
| 5 mg | Beta-Carotin |
| 100 mg | Magnesium |
| 100 mg | Chromhefe |
| 50 mg | Bioflavonoide |

| Kapsel B: | |
|---|---|
| 300 mg | Olivenöl |
| 300 mg | Weizenkeimöl |
| 100 mg | Magnesium |
| 4,5 mg | Vitamin B1 |
| 5 mg | Vitamin B2 |
| 5 mg | Vitamin B6 |
| 8 mg | Pantothensäure |
| 10 mg | Niacin |
| 5 µg | Vitamin B12 |
| 200 mg | Zinkhefe (10 mg Zn/g Hefe) |
| 100 mg | Grüner Tee-Extrakt |

### Beispiel 3

Dieses Beispiel umfaßt ein Zwei-Phasen-Präparat zur zeitlich abgestuften Anwendung, speziell auf den Organismus von Sportlern abgestimmt, als Diätetikum. Die in Tabelle III angegebenen Zusammensetzungen für die Kapsel A und den Beutel B werden durch dem Fachmann geläufige Verfahren erhalten. Zusätzlich kann die Rezeptur bekannte Zusatz-, Hilfs- und Trägerstoffe und Lösungsmittel enthalten.

**Tabelle III**

| Kapsel A: | |
|---|---|
| 200 mg | Magnesium |
| 250 mg | Leinöl |
| 250 mg | Nachtkerzenöl |
| 36 mg | Vitamin E |
| 225 mg | Vitamin C |
| 6 mg | Beta-Carotin |
| 4,5 mg | Vitamin B1 |
| 5 mg | Vitamin B2 |
| 5 mg | Vitamin B6 |
| 8 mg | Pantothensäure |
| 10 mg | Niacin |
| 5 µg | Vitamin B12 |
| 200 mg | Zinkhefe (10 mg Zn/g Hefe) |
| 100 mg | Chromhefe |
| 100 mg | Citrusflavonoide |

| Produkt B (Beutel): | |
|---|---|
| Aminosäure- und Aminosäurederivatemischung mit Vitamin B6 und Coenzym Q₁₀ zur Förderung der Regeneration und des Aufbaustoffwechsels in der Nachtphase, und auch zur Förderung des Fettstoffwechsels/Fettabbaus. | |
| 100 mg | Zinkhefe (10 mg Zn/g Hefe) |
| 100 mg | Magnesium |
| 200 mg | Weizenkeimöl |
| 50 mg | Citrus-Flavonoide |
| 2000 mg | Aminosäure- und Aminosäurederivatemischung mit Vitamin B6 und Coenzym Q₁₀ |

## Patentansprüche

1. Verwendung eines Produkts A und eines Produkts B für die Herstellung eines Nahrungsergänzungsmittel-Zwei-Phasen-Präparats zur zeitlich abgestuften Anwendung, umfassend das Produkt A für die Tagesphase und das Produkt B für die Nachtphase wobei
das Produkt A
a) mindestens einen Vertreter der ungesättigten Fettsäuren und/oder
b) mindestens einen Vertreter aus der Gruppe Spurenelemente und Mineralstoffe und/oder
c) mindestens einen Vertreter aus der Gruppe der Vitamine und/oder
d) mindestens einen Vertreter der bioaktiven Pflanzenstoffe und/oder
e) mindestens eine Aminosäure und/oder ein Aminosäurederivat,
und
das Produkt B
a) mindestens einen Vertreter der ungesättigten Fettsäuren und/oder
b) mindestens einen Vertreter aus der Gruppe Spurenelemente und Mineralstoffe und/oder
c) mindestens einen Vertreter aus der Gruppe der Vitamine und/oder
d) mindestens einen Vertreter der bioaktiven Pflanzenstoffe und/oder
e) mindestens eine Aminosäure und/oder ein Aminosäurederivat,
umfaßt, mit der Maßgabe, daß sich Produkt A und Produkt B in ihrer mengenmäßigen und/oder ihrer stofflichen Zusammensetzung voneinander unterscheiden.

2. Verwendung nach Anspruch 1 umfassend
ein Produkt A und ein Produkt B, enthaltend jeweils unabhängig voneinander die Bestandteile a), b) und c).

3. Verwendung nach Anspruch 1 oder 2, in welchem das Produkt A und/oder das Produkt B zusätzlich Sojalecithin umfaßt.

4. Verwendung nach Anspruch 2 oder 3, in welchem das Produkt A und/oder das Produkt B zusätzlich mindestens einen Vertreter aus der Gruppe der bioaktiven Pflanzenstoffe umfaßt.

5. Verwendung nach Anspruch 2 bis 4, in welchem das Produkt A und/oder das Produkt B zusätzlich eine Aminosäure und/oder ein Aminosäurederivat oder eine Aminosäure- und/oder Aminosäurederivatemischung umfaßt.

6. Verwendung nach Anspruch 2, umfassend ein Produkt A, enthaltend den Bestandteil a) in einer Menge von 5 bis 90 Gew.-%, den Bestandteil b) in einer Menge von 1 bis 80 Gew.-% und den Bestandteil c) in einer Menge von 1 bis 70 Gew.-%, und
ein Produkt B, enthaltend den Bestandteil a) in einer Menge von 1 bis 90 Gew.-%, den Bestandteil b) in einer Menge von 1 bis 80 Gew.-% und den Bestandteil c) in einer Menge von 0,5 bis 50 Gew.-%, bezogen auf die Summe aller Wirkstoffe im Produkt A bzw. B.

7. Verwendung nach Anspruch 2, umfassend ein Produkt A, enthaltend den Bestandteil a) in einer Menge von 30 bis 60 Gew.-%, den Bestandteil b) in einer Menge von 5 bis 30 Gew.-% und den Bestandteil c) in einer Menge von 20 bis 45 Gew.-%, und
ein Produkt B, enthaltend den Bestandteil a) in einer Menge von 5 bis 80 Gew.-%, den Bestandteil b) in einer Menge von 3 bis 60 Gew.-% und den Bestandteil c) in einer Menge von 1 bis 35 Gew.-%, bezogen auf die Summe aller Wirkstoffe im Produkt A bzw. B.

8. Verwendung nach Anspruch 1 oder 2, in welchem das Produkt A und/oder das Produkt B zusätzlich 1 bis 40 Gew.-% Sojalecithin umfaßt, bezogen auf die Summe aller Wirkstoffe im Produkt A bzw. B.

9. Verwendung nach Anspruch 1 oder 2, in welchem das Produkt A und/oder das Produkt B zusätzlich 1 bis 50 Gew.-% mindestens eines Vertreters aus der Gruppe der bioaktiven Pflanzensubstanzen umfaßt, bezogen auf die Summe aller Wirkstoffe im Produkt A bzw. B.

10. Verwendung nach Anspruch 1 oder 2, in welchem die ungesättigten Fettsäuren des Produkts A und des Produkts B die in pflanzlichen und tierischen Ölen enthaltenen ungesättigten Fettsäuren sind.

11. Verwendung nach Anspruch 1 oder 2, in welchem die Spurenelemente und Mineralstoffe des Produkts A und des Produkts B Zink, Magnesium, Calcium, Silicium und Chrom sind.

12. Verwendung nach Anspruch 1 oder 2, in welchem die Vitamine des Produkts A und des Produkts B die Vitamine B1, B2, B6, B12, C, E, Pantothensäure, Niacin, Folsäure und Biotin sind.

13. Verwendung nach Anspruch 1, 4 oder 9, in welchem die bioaktiven Pflanzensubstanzen des Produkts A und/oder des Produkts B die in Karotten-, Brokkoli- und Citrusextrakt und die in grünem Tee enthaltenen Bioflavonoide sind.

14. Verwendung nach Anspruch 1 oder 5, in welchem das Produkt A und/oder das Produkt B Arginin, Ornithin, Leucin, Isoleucin, Valin und Tryptophan als Aminosäuren und/oder Taurin und Carnitin als Aminosäurederivate einzeln oder in Aminosaure- und/oder Aminosäurederivatemischung enthalten.

15. Kit, enthaltend die gleiche Anzahl von Kapseln oder Trinkampullen A und Kapseln oder Trinkampullen B, wobei die Kapseln oder Trinkampullen A ein Produkt A und die Kapseln oder Trinkampullen B ein Produkt B, wie sie in den Ansprüchen 1 bis 14 definiert sind, enthalten.

## Claims

1. Use of a product A and a product B for the preparation of a two-phase food supplement preparation for use at differential times comprising the product A for the day phase and the product B for the night phase wherein
the product A comprises
a) at least one representative of the unsaturated fatty acids and/or
b) at least one representative of the group of trace elements and minerals and/or
c) at least one representative of the group of vitamins and/or
d) at least one representative of the bioactive plant substances and/or
e) at least one amino acid and/or amino acid derivative,
and
the product B comprises
a) at least one representative of the unsaturated fatty acids and/or
b) at least one representative of the group of trace elements and minerals and/or
c) at least one representative of the group of vitamins and/or
d) at least one representative of the bioactive plant substances and/or
e) at least one amino acid and/or amino acid derivative,
with the proviso that product A and product B differ from each other in their quantitative and/or material composition.

2. Use according to claim 1 comprising
a product A and a product B which comprise, independently of each other, the components a), b), and c).

3. Use according to claim 1 or 2, wherein the product A and/or the product B additionally comprise(s) soy lecithin.

4. Use according to claim 2 or 3, wherein the product A and/or the product B additionally comprise(s) at least one representative of the group of bioactive plant substances.

5. Use according to claims 2 to 4, wherein the product A and/or the product B additionally comprise(s) an amino acid and/or amino acid derivative or a mixture of amino acids and/or amino acid derivatives.

6. Use according to claim 2, comprising a product A which comprises the component a) in an amount of from 5 to 90% by weight, the component b) in an amount of from 1 to 80% by weight and the component c) in an amount of from 1 to 70% by weight, and
a product B which comprises the component a) in an amount of from 1 to 90% by weight, the component b) in an amount of from 1 to 80% by weight and the component c) in an amount of from 0.5 to 50% by weight, based on the sum of all active ingredients of product A and B, respectively.

7. Use according to claim 2, comprising a product A which comprises the component a) in an amount of from 30 to 60% by weight, the component b) in an amount of from 5 to 30% by weight and the component c) in an amount of from 20 to 45% by weight, and
a product B which comprises the component a) in an amount of from 5 to 80% by weight, the component b) in an amount of from 3 to 60% by weight and the component c) in an amount of from 1 to 35% by weight, based on the sum of all active ingredients of product A and B, respectively.

8. Use according to claim 1 or 2, wherein the product A and/or the product B additionally comprise(s) 1 to 40% by weight of soy lecithin, based on the sum of all active ingredients of product A and B, respectively.

9. Use according to claim 1 or 2, wherein the product A and/or the product B additionally comprise(s) 1 to 50% by weight of at least one representative of the group of bioactive plant substances, based on the sum of all active ingredients of product A and B, respectively.

10. Use according to claim 1 or 2, wherein the unsaturated fatty acids of the product A and the product B are unsaturated fatty acids contained in vegetal and animal oils.

11. Use according to claim 1 or 2, wherein the trace elements and minerals of the product A and the product B are zinc, magnesium, calcium, silicon and chromium.

12. Use according to claim 1 or 2, wherein the vitamins of the product A and the product B are the vitamins B1, B2, B6, B12, C, E, pantothenic acid, niacin, folic acid and biotin.

13. Use according to claim 1, 4 or 9, wherein the bioactive plant substances of the product A and/or the product B are the bioflavonoids contained in carrot, broccoli and citrus extracts and green tea.

14. Use according to claim 1 or 5, wherein the product A and/or the product B contain arginine, ornithine, leucine, isoleucine, valine and tryptophan as amino acids and/or taurine and carnitine as amino acid derivatives, either individually or as a mixture of amino acids and/or amino acid derivatives.

15. Kit containing the same numbers of capsules or ampoules for drinking A and capsules and ampoules for drinking B, wherein the capsules or ampoules for drinking A contain a product A, and the capsules or ampoules for drinking B contain a product B as defined in claims 1 to 14, respectively.

## Revendications

1. Utilisation d'un produit A et d'un produit B pour la production d'une préparation biphasée de complément nutritif pour une utilisation échelonnée dans le temps, contenant le produit A pour la phase diurne et le produit B pour la phase nocturne, où :
le produit A contient
a) au moins un représentant des acides gras insaturés et/ou
b) au moins un représentant du groupe des oligo-éléments et des minéraux et/ou
c) au moins un représentant du groupe des vitamines et/ou
d) au moins un représentant des matières végétales bioactives et/ou
e) au moins un acide aminé et/ou un dérivé d'acide aminé, et
le produit B contient
a) au moins un représentant des acides gras insaturés et/ou
b) au moins un représentant du groupe des oligo-éléments et des minéraux et/ou
c) au moins un représentant du groupe des vitamines et/ou
d) au moins un représentant des matières végétales bioactives et/ou
e) au moins un acide aminé et/ou un dérivé d'acide aminé, à la condition que le produit A et le produit B se différencient par leur composition en masse et/ou matière.

2. Utilisation selon la revendication 1, comportant
un produit A et un produit B contenant à chaque fois les constituants a), b) et c), indépendamment les uns des autres.

3. Utilisation selon la revendication 1 ou 2, où le produit A et/ou le produit B contient de plus de la lécithine de soja.

4. Utilisation selon la revendication 2 ou 3, où le produit A et/ou le produit B contient de plus au moins un représentant du groupe des matières végétales bioactives.

5. Utilisation selon la revendication 2 à 4, où le produit A et/ou le produit B contient de plus un acide aminé et/ou un dérivé d'acide aminé ou bien un mélange d'acides aminés et/ou de dérivés d'acides aminés.

6. Utilisation selon la revendication 2, comportant un produit A contenant le constituant a) en une quantité de 5 à 90% en poids, le constituant b) en une quantité de 1 à 80% en poids et le constituant c) en une quantité de 1 à 70% en poids, et
un produit B, contenant le constituant a) en une quantité de 1 à 90% en poids, le constituant b) en une quantité de 1 à 80% en poids et le constituant c) en une quantité de 0,5 à 50% en poids, en se rapportant à la somme de tous les agents actifs dans le produit A ou respectivement B.

7. Utilisation selon la revendication 2 comportant un produit A, contenant le constituant a) en une quantité de 30 à 60% en poids, le constituant b) en une quantité de 5 à 30% en poids et le constituant c) en une quantité de 20 à 45% en poids, et
un produit B, contenant le constituant a) en une quantité de 5 à 80% en poids, le constituant b) en une quantité de 3 à 60% en poids et le constituant c) en une quantité de 1 à 35% en poids, en se rapportant à la somme de tous les agents actifs dans le produit A ou respectivement B.

8. Utilisation selon la revendication 1 ou 2, où le produit A et/ou le produit B contient de plus 1 à 40% en poids de lécithine de soja, en se rapportant à la somme de tous les agents actifs dans le produit A ou respectivement B.

9. Utilisation selon la revendication 1 ou 2, où le produit A et/ou le produit B contient de plus 1 à 50% en poids d'au moins un représentant du groupe des substances végétales bioactives, en se rapportant à la somme de tous les agents actifs dans le produit A ou respectivement le produit B.

10. Utilisation selon la revendication 1 ou 2, où les acides gras insaturés du produit A et du produit B sont des acides gras insaturés contenus dans les huiles végétales et animales.

11. Utilisation selon la revendication 1 ou 2, où les oligo-éléments et les minéraux du produit A et du produit B sont le zinc, le magnésium, le calcium, le silicium et le chrome.

12. Utilisation selon la revendication 1 ou 2, où les vitamines du produit A et du produit B sont les vitamines B1, B2, B6, B12, C, E, l'acide pantothénique, la niacine, l'acide folique et la biotine.

13. Utilisation selon la revendication 1, 4 ou 9, où les substances végétales bioactives du produit A et/ou du produit B sont les bioflavonoïdes contenus dans des extraits de carotte, de brocoli et de citrus et contenus dans le thé vert.

14. Utilisation selon la revendication 1 ou 5, où le produit A et/ou le produit B contient de l'arginine, de l'ornithine, de la leucine, de l'isoleucine, de la valine et du tryptophane en tant qu'acides aminés et/ou de la taurine et de la carnitine en tant que dérivés d'acides aminés, individuellement ou en mélange d'acides aminés et/ou de dérivés d'acides aminés.

15. Nécessaire, contenant le même nombre de capsules ou d'ampoules buvables A et de capsules ou d'ampoules buvables B, où les capsules ou ampoules buvables A contiennent un produit A et les capsules ou ampoules buvables B contiennent un produit B tels qu'ils sont définis dans les revendications 1 à 14.
